# EUROPÄISCHE PATENTANMELDUNG

(11) **EP 3 153 191 A1**
(43) Veröffentlichungstag der Anmeldung: **12.04.2017**
(21) Anmeldenummer: 15189242.9
(22) Anmeldetag: 09.10.2015
(51) Int. Cl.: A61M 1/10, A61M 1/12

(54) **BLUTPUMPE**

(71) Anmelder: ECP Entwicklungsgesellschaft mbH, 52074 Aachen (DE)
(72) Erfinder: Schumacher, Jörg, 14513 Teltow (DE); Spanier, Gerd, 52074 Aachen (DE); Sieß, Thorsten, 52074 Aachen (DE)
(74) Vertreter: Pfenning, Meinig & Partner mbB

(57) **Zusammenfassung**

Die Anmeldung betrifft eine Blutpumpe. Die Blutpumpe umfasst eine in einem Katheter (2) geführte und flexible Antriebswelle (3), ein in einem distalen Bereich der Antriebswelle (3) mit der Antriebswelle (3) verbundenes Förderelement (6) und einen Motor (7), wobei der Motor (7) einen Stator (36) und einen in dem Stator (36) drehbar gelagerten Rotor (30) umfasst. Der Stator (36) umfasst eine Wicklung (37) und der Rotor (30) einen Rotormagneten (31). Außerdem ist die Antriebswelle (3) an einem proximalen Ende der Antriebswelle (3) mit dem Rotor (30) verbunden. Der Stator (36) und der Rotor (30) sind unlösbar miteinander verbunden und bilden einen Spalt (40) mit ringförmigem Querschnitt aus, der durch den Rotor (30) und den Stator (36) begrenzt wird.

## Beschreibung

Die Anmeldung betrifft eine Blutpumpe gemäß dem Oberbegriff des Anspruchs 1. Insbesondere betrifft die Anmeldung eine Blutpumpe mit einem Motor.

Aus dem Stand der Technik sind Blutpumpen mit einem proximalen und einem distalen Ende sowie einem dazwischen angeordneten Katheter bekannt, bei denen eine flexible Antriebswelle in einem Innenraum des Katheters geführt wird. Solche Blutpumpen weisen an ihrem distalen Ende typischerweise einen Pumpenkopf auf, der ein faltbares Gehäuse und ein faltbares Förderelement umfasst, wobei das Förderelement mit einem distalen Bereich der Antriebswelle verbunden ist. Derartige Pumpenköpfe können an schwer zugängliche Orte geführt werden. Beispielsweise kann ein solcher Pumpenkopf durch die Femoralarterie über den Aortenbogen in einen Bereich der Aortenklappe eines Patienten eingeschoben werden, um dort Blut vom linken Ventrikel des Herzens in die Aorta zu befördern. Angetrieben wird die Antriebswelle am proximalen Ende der Blutpumpe durch einen Motor, welcher sich typischerweise außerhalb des Körpers des Patienten befindet. Eine derartige Blutpumpe ist beispielsweise in der Druckschrift EP 2 868 331 A2 beschrieben.

Die Druckschrift US 4,895,557 offenbart eine Motoranordnung zum Antrieb einer Blutpumpe. Diese Motoranordnung umfasst ein sterilisierbares und fluiddichtes Rotorgehäuse, in dem sich der Rotor befindet. Das Rotorgehäuse ist eingerichtet, zum Betrieb so in eine Aussparung eines Statorgehäuses geführt zu werden, dass der Rotor von einem Stator umgeben wird. Nach dem Betrieb kann das Rotorgehäuse aus der Aussparung des Statorgehäuses herausgezogen und entsorgt werden.

Ein Nachteil einer solchen Motoranordnung ist, dass diese vergleichsweise großvolumig ist, was insbesondere bei einer Befestigung der Motoranordnung an einem Bein eines Patienten Probleme verursachen kann. Darüber hinaus kann eine derartige Motoranordnung im Betrieb zu einer beträchtlichen ungewünschten Wärmeentwicklung führen. Ein weiterer Nachteil ist, dass es bei einer Montage des Systems zu erheblichen Verschmutzungen des Stators kommen kann, beispielsweise durch Unreinheiten an Handschuhen eines Anwenders, was gegebenenfalls eine aufwendige Reinigung und Sterilisation des wiederverwendbaren Stators erfordert.

Der vorliegenden Anmeldung liegt die Aufgabe zugrunde, eine Blutpumpe vorzuschlagen, die einfach in der Handhabung ist und die die oben genannten Nachteile bekannter Vorrichtungen überwindet.

Diese Aufgabe wird gelöst durch eine Blutpumpe mit den Merkmalen des Hauptanspruchs. Vorteilhafte Weiterbildungen ergeben sich mit den Merkmalen der abhängigen Ansprüche und der Ausführungsbeispiele.

Die vorgeschlagene Blutpumpe umfasst eine in einem Katheter geführte und flexible Antriebswelle, ein in einem distalen Bereich der Antriebswelle mit der Antriebswelle verbundenes Förderelement und einen Motor, wobei der Motor einen Stator und einen in dem Stator drehbar gelagerten Rotor umfasst. Der Stator umfasst eine Wicklung und der Rotor einen Rotormagneten. Außerdem ist die Antriebswelle an einem proximalen Ende der Antriebswelle mit dem Rotor verbunden. Der Stator und der Rotor sind unlösbar miteinander verbunden und bilden einen Spalt aus, der durch den Rotor und den Stator begrenzt wird.

Insbesondere gegenüber den aus dem Stand der Technik bekannten modularen Bauweisen von Motoren für Blutpumpen, bei denen der Rotor und der Stator für einen Anwender lösbar ausgeführt sind, erlaubt die vorgeschlagene Blutpumpe eine kompakte Bauweise. Bei dieser bilden der Stator und der Rotor eine Einheit, die beispielsweise kraft- oder stoffschlüssig miteinander verbunden sein kann. Durch die kompakte Bauweise kann ein geringes Gewicht des Motors erreicht werden, wodurch ein gegebenenfalls an einem Patientenbein befestigter Motor eine geringere Last darstellt.

Die Wicklung kann einen Innenradius aufweisen, der maximal einem 1,5-fachen, bevorzugt maximal einem 1,25-fachen, besonders bevorzugt maximal einem 1,15-fachen, eines Außenradius des Rotormagneten entspricht. Durch den Abstand zwischen der Wicklung und dem Rotormagneten ist der magnetische Luftspalt gegeben. Ein geringer Abstand zwischen Rotormagnet und Wicklung erlaubt eine effiziente Umwandlung von elektrischer Leistung in Pumpleistung, so dass Wärmeverluste im Motor beim Betrieb mit einer gewünschten Pumpleistung gering gehalten werden können. Da bei dem vorgeschlagenen Motor durch die einteilige Bauweise keine Gehäuseteile in dem magnetischen Luftspalt vorgesehen sein müssen, kann ein geringer Abstand zwischen der Wicklung und dem Rotormagneten im Vergleich zu Bauformen erreicht werden, bei denen der Stator und der Rotor einzeln gehäust ausgeführt sind. Bei einem Innenradius der Wicklung von 6 mm kann der Außenradius des Rotormagneten beispielsweise mehr als 5,25 mm betragen.

Beispielsweise kann ein radialer Abstand zwischen der Wicklung und dem Rotormagneten maximal 2 mm, vorzugsweise maximal 1,25 mm, besonders vorzugsweise maximal 0,75 mm, betragen.

Der Spalt weist typischerweise einen ringförmigem Querschnitt auf. Der Spalt weist eine Breite auf, die einer Breite des magnetischen Luftspalts entspricht oder die geringer ist als die Breite des magnetischen Luftspalts. Es kann vorgesehen sein, dass der Spalt eine Breite von maximal 1 mm, vorzugsweise maximal 0,5 mm, besonders vorzugsweise maximal 0,25 mm, aufweist.

Außerdem kann eine Spülöffnung vorgesehen sein, die in fluider Verbindung mit dem Spalt steht. Die Spülöffnung kann in fluider Verbindung mit einem Spülanschluss stehen. Ein solcher Spülanschluss kann beispielsweise an einem proximalen Ende des Motors angeordnet sein.

Darüber hinaus kann es vorgesehen sein, dass der Spalt in fluider Verbindung mit einem zwischen dem Katheter und der Antriebswelle ausgebildeten Zwischenraum steht. Auf diese Weise kann durch den Spülanschluss eine Spülflüssigkeit durch den Spalt in den Zwischenraum zwischen der Antriebswelle und dem Katheter gespült werden. Dadurch kann ein Schmieren der Antriebswelle in dem Katheter erreicht werden. Darüber hinaus kann durch ein Einleiten der Spülflüssigkeit durch den Spülanschluss verhindert werden, dass Blut eines Patienten in den Motor und insbesondere in den Spalt gelangt. Es kann auch eine Spülflüssigkeit durch den Spülanschluss, den Spalt und den Zwischenraum zwischen dem Katheter und der Antriebswelle in den Körper des Patienten geleitet werden. Als Spülflüssigkeit kann beispielsweise eine Glukoselösung verwendet werden.

Es kann vorgesehen sein, dass der Rotor von einem proximalen Ende zu einem distalen Ende von der Spülflüssigkeit umspült wird. Es kann auch vorgesehen sein, dass der Rotor von seinem distalen zu seinem proximalen Ende von der Spülflüssigkeit umspült wird.

Es können auch Kathether mit mehreren Lumen Verwendung finden, so dass eine Hin- und eine Rückspülung erreicht werden kann, wie es beispielsweise in der Druckschrift US 4,895,557 beschrieben ist. Hierbei können zwei oder mehrere Anschlüsse für die Spülflüssigkeit an dem Motor vorgesehen sein.

Es kann vorgesehen sein, dass der Spalt eine minimale Breite von 0,05 mm aufweist, um einen zuverlässigen Durchfluss der Spülflüssigkeit durch den Spalt zu gewährleisten.

Es kann vorgesehen sein, dass die Wicklung in eine Vergussmasse eingegossen ist. Ein Vergießen der Wicklung mit einer Vergussmasse ist geeignet, gegebenenfalls vorhandene Vertiefungen an einer Oberfläche der Wicklung zu verschließen und zu nivellieren. Die Vergussmasse kann ein dünnviskoses Material umfassen, welches geeignet ist, in die Vertiefungen hineinzufließen und diese auszufüllen.

Es kann vorgesehen sein, dass die Vergussmasse einen den Spalt begrenzenden Teil des Stators bildet. Durch die Vergussmasse kann eine weitgehend glatte Begrenzungsfläche des Spalts erreicht werden. Die Vergussmasse kann beispielsweise Epoxidharz umfassen. Darüber hinaus kann vorgesehen sein, dass die Vergussmasse beispielsweise Aluminiumoxid, Eisenpulver oder andere wärmeleitende Substanzen für eine verbesserte Wärmeübertragung aufweist. Zusätzlich kann durch die Vergussmasse erreicht werden, dass nach einem Entlüften des Spaltes eine verminderte Anzahl von Luftbläschen an dem Stator anhaftet.

Durch die Vergussmasse kann eine Beschädigung oder Korrosion der Wicklung durch eine Spülflüssigkeit oder gegebenenfalls durch die Spülflüssigkeit transportierte Partikel verhindert werden. Darüber hinaus kann durch die Vergussmasse verhindert werden, dass sich die Partikel an der Wicklung absetzen.

Außerdem kann es vorgesehen sein, dass die Vergussmasse biokompatibles Material aufweist. Typischerweise ist die Vergussmasse hierbei vollständig aus biokompatiblem Material gefertigt, damit keine toxischen Substanzen über den Spülanschluss an den Patienten abgegeben werden. Es kann beispielsweise auch vorgesehen sein, dass die Wicklung mit Parylene beschichtet ist.

Es kann auch vorgesehen sein, dass der Stator eine fluiddichte Hülse mit im Wesentlichen ringförmigem Querschnitt aufweist, durch die der Spalt begrenzt wird. Durch die Hülse kann beispielsweise die Wicklung des Stators von der Spülflüssigkeit getrennt werden. Dadurch kann eine Beschädigung der Wicklung durch die Spülflüssigkeit verhindert werden. Es kann vorgesehen sein, dass die Hülse einen den Spalt begrenzenden Teil des Stators bildet.

Die Hülse kann auch derart ausgeführt sein, dass zusätzlich zur Wicklung auch andere Motorteile durch die Hülse von der Spülflüssigkeit getrennt und somit vor einer Beschädigung bewahrt werden. Beispielsweise können gegebenenfalls im Motor befindliche Lötstellen durch die Hülse vor einer Korrosion geschützt werden.

Die Hülse kann beispielsweise einen Kunststoff, insbesondere Polyetheretherketon oder Polyethylen, oder Glas umfassen. Es kann auch vorgesehen sein, dass die Hülse aus einem elastischen Kunststoff, der beispielsweise Polyethylen umfasst, ausgebildet ist. Die Hülse dient einem Führen der Spülflüssigkeit und dient nicht notwendigerweise einer mechanischen Stabilisierung des Motors. Daher ist es möglich, dass die Hülse dünnwandig und/oder aus einem biegbaren Material gefertigt ist. Darüber hinaus wird eine äußere Form des Motors oder eines Teils des Motors nicht durch die Form der Hülse bestimmt. Daher kann es vorteilhaft sein, dass die Hülse die Wicklung und/oder den Rotor in axialer Richtung nur geringfügig überdeckt. Es kann beispielsweise vorgesehen sein, dass die Hülse eine Ausdehnung in axialer Richtung aufweist, die geringer als das 1,5-fache einer axialen Ausdehnung des Rotormagneten ist.

Es kann vorgesehen sein, dass der Rotor durch mindestens ein Gleitlager radial gelagert ist. Beispielsweise kann der Rotor durch zwei Gleitlager gelagert sein. Das mindestens eine Gleitlager kann beispielsweise nichtmagnetisierbare Materialien und/oder keramische Materialien, insbesondere Aluminiumoxid, Zirconiumoxid, Yttrium-stabilisiertes Zirconiumoxid oder Siliciumnitrid, umfassen. Darüber hinaus kann das Gleitlager beispielsweise Stahl, insbesondere Implantatstahl, umfassen. Es kann beispielsweise auch eine biokompatible Beschichtung mit diamantähnlichem amorphem Kohlenstoff vorgesehen sein.

Außerdem kann der Rotor durch mindestens ein Kugellager radial gelagert sein. Es kann vorgesehen sein, dass das mindestens eine Kugellager nichtmagnetisierbares Material umfasst. Insbesondere kann es vorgesehen sein, dass Teile des Kugellagers, an denen durch einen Betrieb des Motors ein Abrieb auftritt, nichtmagnetisierbares Material umfassen oder aus nichtmagnetisierbarem Material gefertigt sind. Dadurch wird erreicht, dass abgeriebenes Material der Teile des Kugellagers nicht an ferromagnetischen Komponenten des Motors anhaftet. Beispielsweise kann verhindert werden, dass ferromagnetischer Abrieb an dem Rotor haften bleibt und dadurch zu Beschädigungen des Rotors führt. Des Weiteren kann verhindert werden, dass ferromagnetischer Abrieb die Wicklung oder andere Komponenten des Motors beschädigt.

Beispielsweise kann das mindestens eine Kugellager Kugeln aufweisen, die ein keramisches Material umfassen. Außerdem kann das mindestens eine Kugellager einen Käfig aufweisen, der einen Kunststoff umfasst. Beispielsweise kann der Käfig Polyethylen oder Polytetrafluorethylen umfassen. Die Kugeln können auch vollständig aus einem keramischen Material bestehen. Der Käfig kann vollständig aus einem Kunststoff ausgebildet sein.

Typischerweise weist der Rotor eine Beschichtung und/oder eine Abdeckung zum Schutz des Rotormagneten auf. Es kann sein, dass die Beschichtung und/oder die Abdeckung einen den Spalt begrenzenden Teil des Rotors ausbildet. Sowohl die Beschichtung als auch die Abdeckung können biokompatibles Material umfassen oder aus biokompatiblem Material bestehen. Beispielsweise kann eine Beschichtung mit Parylene oder einem biokompatiblen Epoxidharz vorgesehen sein. Es kann auch eine Beschichtung aus diamantähnlichem amorphem Kohlenstoff vorgesehen sein. Die Beschichtung kann eine Dicke aufweisen, die geringer als 100 µm, vorzugsweise geringer als 10 µm, ist. Der Rotor kann beispielsweise eine Abdeckung aus Polyetheretherketon oder Edelstahl aufweisen.

Die Blutpumpe kann einen entfaltbaren Pumpenkopf aufweisen, der das Förderelement und ein Gehäuse umfasst, wobei das Förderelement und das Gehäuse derart ausgebildet sind, dass diese sich nach einer aufgezwungenen Kompression selbstständig entfalten. Ein entfaltbarer Pumpenkopf erlaubt eine relativ große Ausführung des Pumpenkopfs und der Förderelemente bei relativ kleinem Durchmesser einer für das Einführen der Blutpumpe vorgesehenen Öffnung im Gewebe eines Patienten.

Typischerweise ist die Pumpe eingerichtet zum Pumpen von Blut von einem Ventrikel in ein Blutgefäß eines Patienten, wenn der Motor außerhalb eines Körpers des Patienten angeordnet ist. Der Motor kann beispielsweise für ein Befestigen an einem Oberschenkel des Patienten eingerichtet sein.

Dafür weist die flexible Antriebswelle eine ausreichende Länge auf, die von der Anatomie des Patienten abhängig ist. Typischerweise weist die flexible Welle hierbei eine Länge von mindestens 50 cm, vorzugsweise mindestens 90 cm, auf. Eine maximale Länge der flexiblen Antriebswelle beträgt 200 cm, vorzugsweise 150 cm.

Bei einer Verwendung einer Blutpumpe, die durch einen Motor angetrieben wird, der sich außerhalb eines Patienten befindet, müssen gegebenenfalls im Vergleich zu Blutpumpen, die durch einen Motor im Patientenkörper angetrieben werden, höhere Anforderungen an die Effizienz des Motors erfüllt werden. Beispielsweise ist für einen Motor, der innerhalb des Patientenkörpers angeordnet ist, der Abtransport von im Betrieb erzeugter Wärme über das Blutsystem des Patienten von Vorteil. Eine von einer außerhalb des Patientenkörpers erzeugte Wärme benötigt hingegen unter Umständen weitere Elemente zur Wärmeabfuhr oder eine besonders effiziente Betriebsweise.

Die Anmeldung betrifft ferner ein Betriebsverfahren für die vorgeschlagene Blutpumpe. Hierbei erwärmt sich eine berührbare Oberfläche eines Gehäuses des Motors während eines Permanentbetriebs bei einer Drehzahl von 15.000, bevorzugt mindestens 30.000, Umdrehungen pro Minute auf eine Temperatur von nicht mehr als 60 °C, vorzugsweise von nicht mehr als 48 °C, besonders vorzugsweise von nicht mehr als 43 °C. insbesondere bei einer Befestigung des Motors an einem Oberschenkel eines Patienten ist es wichtig, dass sich das Gehäuse des Motors im Betrieb nicht zu stark erhitzt.

Darüber hinaus kann ein Betriebsverfahren vorgesehen sein, bei dem sich berührbare Oberflächen des Gehäuses des Motors während eines Permanentbetriebs der Blutpumpe bei einer Förderleistung von mindestens 1 l/min, bevorzugt mindestens 2 l/min, auf eine Temperatur von nicht mehr als 60 °C, vorzugsweise von nicht mehr als 48 °C, besonders vorzugsweise von nicht mehr als 43 °C.

Es kann vorgesehen sein, dass die Blutpumpe zum Abführen von im Betrieb verursachter Wärme einen Kühlkörper, beispielsweise mit Kühlrippen, die mit dem Motor wärmeleitend verbunden sind, oder ein Wärmerohr aufweist. Es kann auch vorgesehen sein, dass die Blutpumpe für eine Wärmeabgabe an ein Gewebe des Patienten, beispielsweise über die Haut des Oberschenkels, eingerichtet ist.

Die Blutpumpe kann mit allen beschriebenen Komponenten steril verpackt geliefert werden. Die Blutpumpe kann beispielsweise mittels Gammasterilisation oder unter Verwendung von Ethylenoxid sterilisiert werden. Nach einem Gebrauch der Blutpumpe kann diese vollständig entsorgt werden. Somit entfällt bei der vorgeschlagenen Blutpumpe eine wiederholte Reinigung oder Sterilisation von Teilen der Blutpumpe, insbesondere des Motors, durch einen Anwender.

Ausführungsbeispiele der Erfindung werden nachfolgend anhand der Abbildungen beschrieben. Es zeigen
- Fig. 1: eine schematische Darstellung einer Pumpenanordnung,
- Fig. 2: eine schematische Darstellung eines Pumpenkopfes,
- Fign. 3(a), (b): zwei weitere schematische Darstellungen des Pumpenkopfes,
- Fig. 4: eine schematische Darstellung eines Gehäuses,
- Fig. 5: eine schematische Darstellung eines Motors und
- Fig. 6: eine schematische Darstellung eines weiteren Motors.

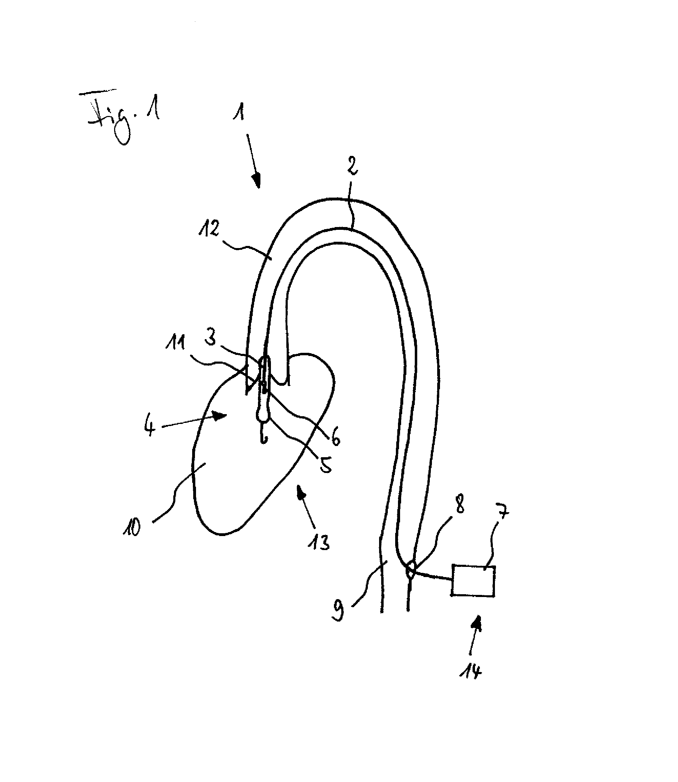

FIG. 1 zeigt schematisch eine Pumpenanordnung 1. Die Pumpenanordnung 1 umfasst einen Katheter 2, in welchem eine flexible Antriebswelle 3 geführt wird. Der Katheter 2 ist mit einem Pumpenkopf 4 verbunden. Dieser Pumpenkopf 4 umfasst ein Gehäuse 5 und ein in dem Gehäuse 5 angeordnetes Förderelement 6, welches über die Antriebswelle 3 mit einem am proximalen Ende der Antriebswelle 3 angeschlossenen Motor 7 angetrieben werden kann. Der Pumpenkopf 4 sowie der Katheter 2 und die Antriebswelle 3 werden über eine Schleuse 8 in die Femoralarterie 9 eingeführt, derart, dass sich der Pumpenkopf 4 im Bereich der linken Herzkammer 10 im Bereich der Aortenklappe 11 befindet. Im Betrieb wird die Antriebswelle 3 durch den Motor 7 angetrieben und die Pumpenanordnung 1 fördert Blut von der linken Herzkammer 10 in die Aorta 12. In der gezeigten Anordnung zur Linksherz-Unterstützung entspricht eine Förderrichtung der Pumpenanordnung 1 der Richtung von einem distalen Ende 13 der Pumpenanordnung 1 zu einem proximalen Ende 14 der Pumpenanordnung 1.

Die Pumpenanordnung 1 kann aber auch eingerichtet sein für ein Fördern von Blut in einer Richtung von dem proximalen Ende 14 zum distalen Ende 13 der Pumpenanordnung 1, was sich beispielsweise für eine Rechtsherz-Unterstützung eignet.

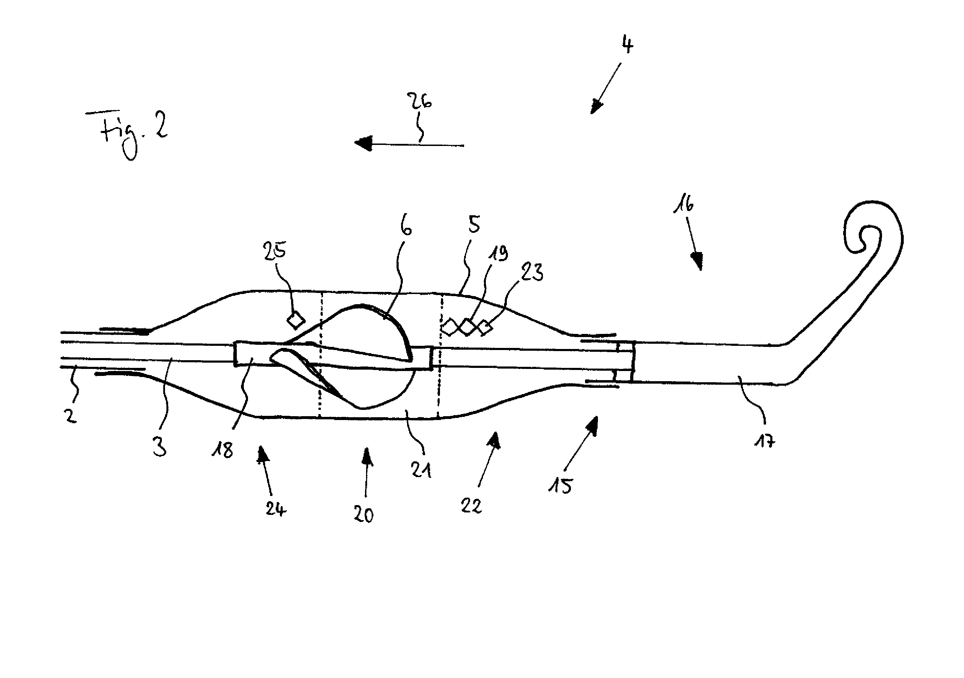

Der Pumpenkopf 4 ist in Fig. 2 schematisch dargestellt. Wiederkehrende Merkmale sind in dieser und in den nachfolgenden Abbildungen mit den gleichen Bezugszeichen versehen. Der Pumpenkopf 4 umfasst das Förderelement 6 und das Gehäuse 5. Das Förderelement 6 ist im vorliegenden Beispiel als Pumpenrotor mit zwei flexiblen Segmenten in Form von Rotorblättern ausgeführt. Zusätzlich ist die Antriebswelle 3 dargestellt, die an einem distalen Bereich 15 des Pumpenkopfes 4 gelagert ist. An einem distalen Ende 16 des Pumpenkopfes 4 ist ein sogenannter Pig-Tail 17 vorgesehen, der aus einem elastisch verformbaren Material gefertigt ist. Mit der Antriebswelle 3 ist ein zylindrisches Element 18 starr verbunden. Auf dem zylindrischen Element 18 ist das Förderelement 6 befestigt. Sowohl das Förderelement 6 als auch das Gehäuse 5 sind derart entfaltbar ausgeführt, dass diese sich nach einer aufgezwungenen Kompression selbstständig entfalten. Das Förderelement 6 ist aus einem Kunststoff gefertigt. Das Gehäuse 5 ist aus dem Formgedächtnismaterial Nitinol gefertigt. Dadurch, dass sowohl das Förderelement 6 als auch das Gehäuse 5 entfaltbar ausgebildet sind, ist der gesamte Pumpenkopf 4 entfaltbar.

Das Gehäuse 5 ist als Rautengitter 19 ausgeführt und weist in einem flüssigkeitsdichten Bereich 20 eine elastische Bespannung 21 aus Polyurethan auf. Die elastische Bespannung 21 bedeckt eine Innenseite und eine Außenseite des Rautengitters 19 derart, dass durch das Gitter 19 ausgebildete rautenförmige Gitteröffnungen in dem flüssigkeitsdichten Bereich 20 durch die elastische Bespannung 21 flüssigkeitsdicht verschlossen werden.

Darüber hinaus weist das Gehäuse 5 einen Einlassbereich 22 auf, der nicht von der elastischen Bespannung 21 bedeckt wird. In dem Einlassbereich 22 bilden die rautenförmigen Gitteröffnungen Einlassöffnungen aus, von denen eine in Fig. 2 beispielhaft mit dem Bezugszeichen 23 versehen ist. Außerdem weist das Gehäuse 5 einen Auslassbereich 24 auf, der ebenfalls nicht von der elastischen Bespannung 21 bedeckt wird. In dem Auslassbereich 24 bilden die rautenförmigen Gitteröffnungen Auslassöffnungen aus, von denen eine beispielhaft gezeigt und mit dem Bezugszeichen 25 versehen ist.

Bei einem Betrieb der Pumpenanordnung 1 wird die Antriebswelle 3 durch den Motor 7 angetrieben, so dass das mit der Antriebswelle 3 verbunden Förderelement 6 um eine Achse der Antriebswelle 3 rotiert. Dadurch wird Blut durch die Einlassöffnungen des Einlassbereichs 22 in das Gehäuse 5 befördert und tritt anschließend durch die Auslassöffnungen des Auslassbereichs 24 aus dem Gehäuse 5 aus. Auf diese Weise wird durch die Pumpenanordnung 1 Blut in einer Förderrichtung 26 gefördert.

Die elastische Bespannung 21 umgibt eine axiale Ausdehnung des Förderelements 6 nicht vollständig. Das Förderelement 6 ragt stattdessen teilweise in den Auslassbereich 24 hinein, so dass zumindest die Auslassöffnung mit dem Bezugszeichen 25 seitlich, d.h. in radialer Richtung, neben dem Förderelement 6 angeordnet ist. Die elastische Bespannung 21 ist hingegen an ihrem distalen Ende derart ausgeführt, dass das Förderelement 6 nicht oder nicht wesentlich in den Einlassbereich 22 hineinragt und somit nicht seitlich von Einlassöffnungen umgeben ist.

Die elastische Bespannung 21 und das Förderelement 6 sind so ausgestaltet und zueinander angeordnet, dass etwa ein Drittel der axialen Ausdehnung des Förderelements 6 nicht von der elastischen Bespannung 21, die den flüssigkeitsdichten Bereich 20 bildet, umgeben ist. Derselbe Anteil der axialen Ausdehnung des Förderelements 6 ist in dem gezeigten Beispiel von dem Auslassbereich 24 umgeben.

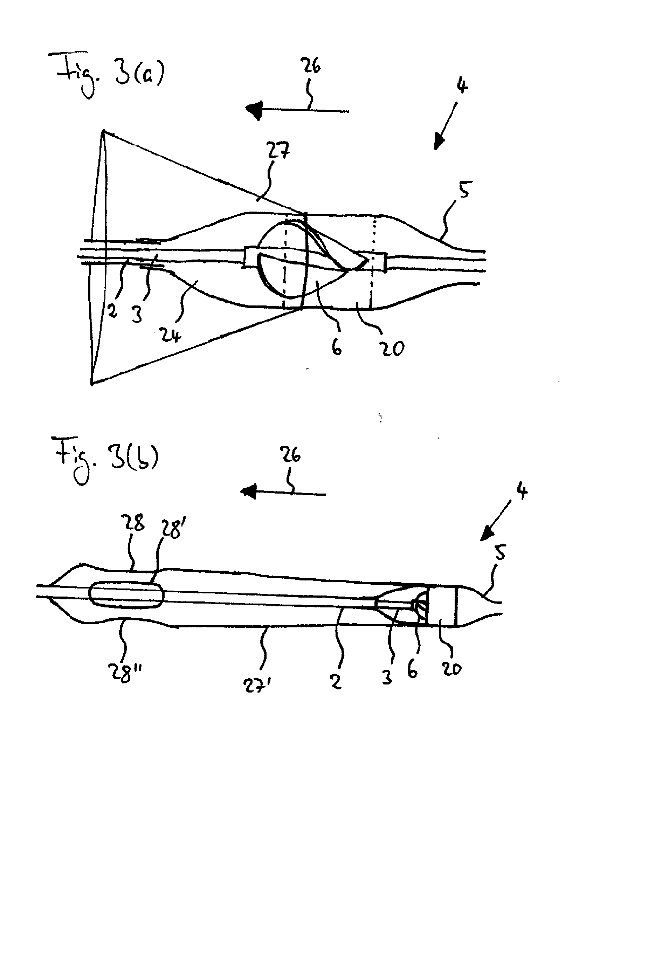

Zusätzlich umfasst der Pumpenkopf 4 ein Abströmelement. Dieses kann als Abströmschirm 27, wie in Fig. 3(a) dargestellt ist, oder als Abströmschlauch 27', wie in Fig. 3(b) dargestellt ist, ausgeführt sein.

Der in Fig. 3(a) dargestellte Abströmschirm 27 ist im flüssigkeitsdichten Bereich 20 des Gehäuses 5 am Gehäuse 5 befestigt. Der Abströmschirm 27 hat die Form eines Mantels eines Kegelstumpfes und erstreckt sich so in Förderrichtung 26, dass dieser in Förderrichtung 26 aufgeweitet ist. Durch den Abströmschirm 27 werden das Förderelement 6 und der Auslassbereich 24 umgeben. Es kann in einer anderen Ausführung auch vorgesehen sein, dass der Auslassbereich 24 durch den Abströmschirm 27 teilweise umgeben wird.

Der Pumpenkopf 4 in Fig. 3(b) unterscheidet sich von dem in Fig. 3(a) gezeigten Pumpenkopf 4 lediglich dadurch, dass anstatt des Abströmschirms 27 ein Abströmschlauch 27' vorgesehen ist. Dieser ist an dem Gehäuse 5 in dem flüssigkeitsdichten Bereich 20 befestigt und erstreckt sich von dort in Förderrichtung 26. Der Abströmschlauch 27' ist aus Polyurethan gefertigt und weist Öffnungen 28, 28', 28" in einem in Förderrichtung 26 gelegenen Bereich auf. In dem gezeigten Beispiel ist der Auslassbereich 24 vollständig von dem Abströmschlauch 27' umgeben. Der Abströmschlauch 27' ist flexibel und verschließt sich selbstständig, wenn eine Blutströmung entgegengesetzt zur Förderrichtung 26 auftritt, dadurch, dass der Abströmschlauch 27' an den Katheter 2 und/oder an das Gehäuse 5 gedrückt wird.

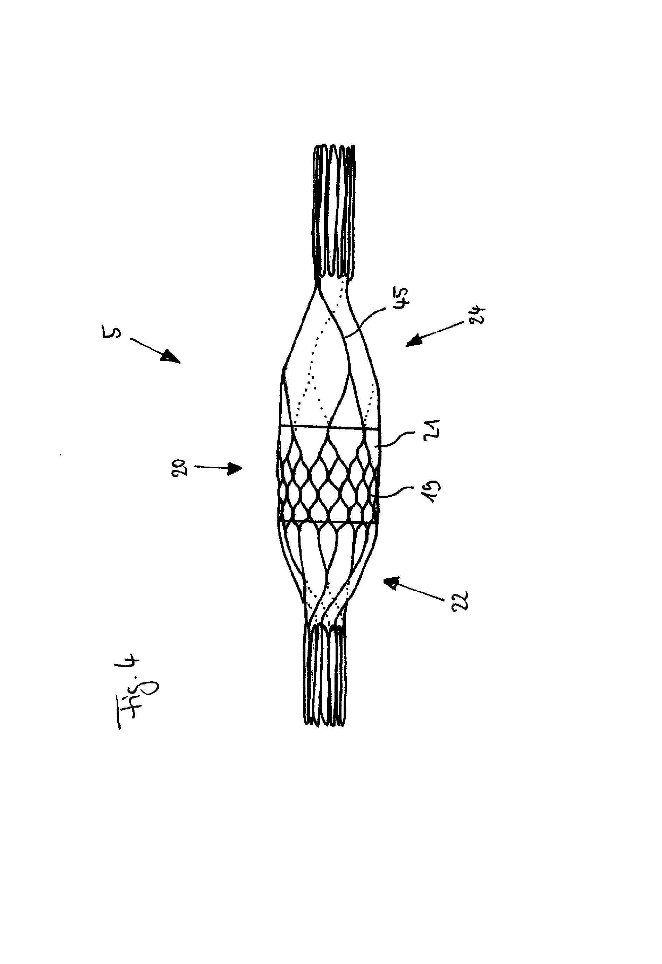

Fig. 4 zeigt schematisch das Rautengitter 19 des Gehäuses 5. Zusätzlich ist der flüssigkeitsdichte Bereich 20 mit der elastischen Bespannung 21 dargestellt sowie der Einlassbereich 22 und der Auslassbereich 24. Bereiche des Einlassbereichs 22 und des Auslassbereichs 24 weisen eine konische Form auf, während der flüssigkeitsdichte Bereich 20 im Wesentlichen rohrförmig ist. Das Gitter 19 weist Gitterstreben auf, von denen eine beispielhaft mit der Bezugsziffer 45 gekennzeichnet ist. Die Gitterstreben 45 verlaufen derart, dass die im Wesentlichen rautenförmigen Gitteröffnungen sowohl im Einlassbereich 22 als auch im Auslassbereich 24 größer sind als im flüssigkeitsdichten Bereich 20. Zur verbesserten Übersicht sind auf einer vom Betrachter abgewandten Seite des Gehäuses 5 angeordnete Gitterstreben in Fig. 4 lediglich gepunktet angedeutet.

Im flüssigkeitsdichten Bereich 20 bilden die Gitterstreben 45 ein vergleichsweise engmaschiges Gitter 19 aus. Entlang eines Umfangs des Gehäuses 5 im flüssigkeitsdichten Bereich 20 weist das Gitter 19 zweiunddreißig Streben beziehungsweise, sofern der Umfang an einer axialen Position des Gehäuses 5 mit Knotenpunkten betrachtet wird, sechzehn Knoten auf. Durch ein derart engmaschiges Gitter 19 wird ein weitgehend runder Querschnitt des Gehäuses 5 in dem flüssigkeitsdichten Bereich 20 erreicht.

Vom flüssigkeitsdichten Bereich 20 in Richtung des Einlassbereichs 22 und in Richtung des Auslassbereichs 24 wird die Anzahl der Gitterstreben 45 entlang eines Umfangs des Gehäuses 5 durch ein paarweises Zusammenführen der Gitterstreben halbiert, so dass das Gehäuse 5 in den entsprechenden Bereichen sechzehn Gitterstreben 45 entlang des Umfangs aufweist, in dem keine Knotenpunkte vorliegen. Anschließend wird die Anzahl der Gitterstreben 45 in Richtung des Einlassbereichs 22 und des Auslassbereichs 24 erneut durch das paarweise Zusammenführen der Gitterstreben 45 reduziert, so dass Gehäuse 5 in diesen Bereichen acht Gitterstreben 45 aufweist. Im Auslassbereich 24 erfolgt eine weitere Reduzierung der Anzahl der Gitterstreben 45 in der oben genannten Weise, so dass das Gehäuse 5 in einem weiter in Förderrichtung 26 gelegenen Bereich lediglich vier Gitterstreben 45 entlang eines Umfangs aufweist.

Durch die beschriebene Reduzierung der Anzahl der Gitterstreben 45 bildet sich in dem Einlassbereich 22 und in dem Auslassbereich 24 ein Gitter 19 mit größeren Gitteröffnungen als im flüssigkeitsdichten Bereich 20 aus.

Die Gitterstreben 45 bilden in den konischen Bereichen des Auslassbereichs 24 und des Einlassbereichs 22 eine spiralförmige Struktur, was bei einem Hinausschieben des Pumpenkopfes 4 aus einer Kanüle zu einem zu einem zuverlässigen Entfalten des Pumpenkopfs 4 führt.

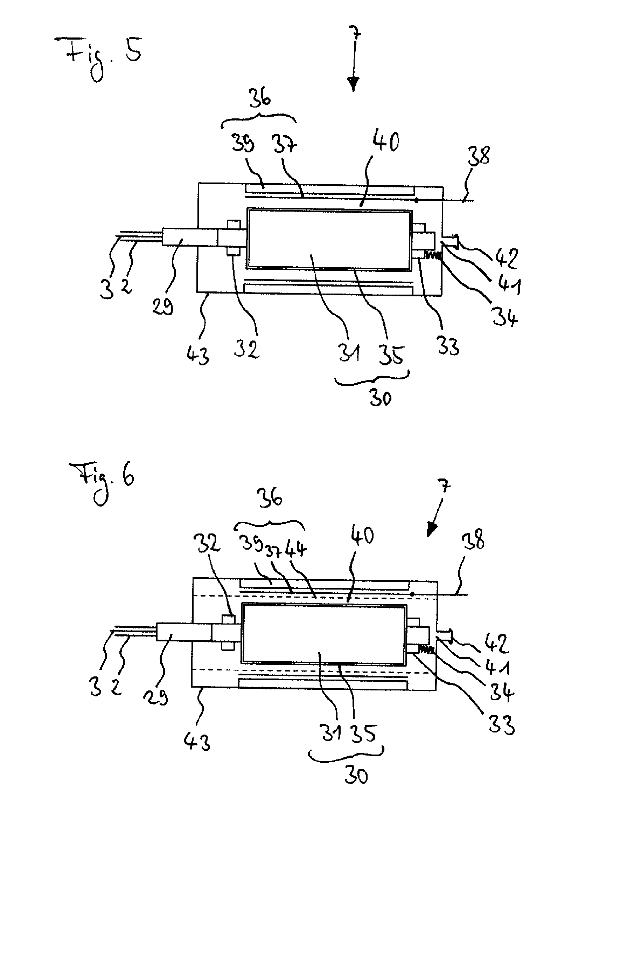

Fig. 5 zeigt eine schematische Ansicht des Motors 7. Der Motor 7 ist im Bereich eines Wellenstummels 29 mit dem Katheter 2 verbunden, der in den Wellenstummel 29 eingeklebt ist. In dem Katheter 2 wird die flexible Antriebswelle 3 geführt. Der Motor 7 weist außerdem einen Rotor 30 auf, der einen Rotormagneten 31 umfasst.

Die flexible Antriebswelle 3 ist derart mit dem Rotor 30 verbunden, dass ein Drehmoment bei einer Rotation des Rotors 30 von dem Rotor 30 auf die flexible Antriebswelle 3 übertragen wird. Über die flexible Antriebswelle wird das Drehmoment auf das Förderelement 6 übertragen, so dass die Pumpenanordnung 1 durch den Motor 7 angetrieben wird.

Der Rotor 30 ist axial durch zwei Lager 32, 33 gelagert. Eines dieser Lager 33 ist durch ein Federelement 34 vorgespannt für eine axiale Stabilisierung des Rotors 30. Das Federelement 34 kann beispielsweise als eine Schraubenfeder oder als eine Ringfeder ausgeführt sein. Die Lager 32, 33 können jeweils als Kugellager oder als Gleitlager ausgeführt sein. Wenn die Lager 32, 33 als Kugellager ausgeführt sind, umfassen die Lager 32, 33 Kugeln aus Keramik und Käfige aus Kunststoff, so dass die Kugellager nichtmagnetisierbares Material aufweisen. Die Ringe der Lager können beispielsweise aus einem magnetisierbaren Metall oder aus einem nichtmagnetisierbaren Material gefertigt sein. Wenn die Lager 32, 33 als Gleitlager ausgeführt sind, umfassen diese jeweils Gleitpartner aus DLC-beschichtetem Implantatstahl und Yttrium-stabilisiertem Zirconiumoxid.

Der Rotormagnet 31 weist eine biokompatible DLC-Beschichtung 35 auf. Außerdem weist der Motor 7 einen Stator 36 auf. Der Stator 36 umfasst mehrere Wicklungen 37, die mit Stromanschlüssen 38 elektrisch leitfähig verbunden sind. Darüber hinaus weist der Stator 36 Rückschlussbleche 39 auf. Die Wicklungen 37 sind mit einem biokompatiblen Epoxidharz vergossen, welches wärmeleitendes Aluminiumoxid enthält.

Zwischen einer Innenseite der Beschichtung der Wicklungen 37 und einer Außenseite der Beschichtung 35 des Rotormagneten 31 ist ein Spalt 40 mit ringförmigem Querschnitt ausgebildet. Der Spalt 40 weist eine Breite von 0,2 mm auf. Dieser Spalt 40 steht in fluider Verbindung mit einer Spülöffnung 41, die mit einem Spülanschluss 42 verbunden ist, wobei der Spülanschluss 42 an einem proximalen Ende des Motors 7 angeordnet ist. Darüber hinaus steht der Spalt 40 in fluider Verbindung mit einem zwischen der Antriebswelle 3 und dem Katheter 2 ausgebildeten Zwischenraum. Somit kann beispielsweise eine Glukoselösung über den Spülanschluss 42 durch die Spülöffnung 41 und den Spalt 40 und den Zwischenraum gespült werden. Auf diese Weise wird der Rotor 30 im Betrieb von der Glukoselösung umspült. Ein radialer Abstand zwischen einer Außenseite des Rotormagneten 31 und einer Innenseite der Wicklungen 37 beträgt 0,5 mm. Ein Innenradius der Wicklungen 37 entspricht hierbei dem 1,1-fachen eines Außenradius des Rotormagneten 31.

Der Stator 36 und der Rotor 30 sind für einen Anwender nicht lösbar miteinander verbunden und in ein Motorgehäuse 43 eingefasst. Das Motorgehäuse 43 kann beispielsweise mit einem Griff oder einem Kühlkörper verbunden sein. Durch den geringen Abstand zwischen den Wicklungen 37 und dem Rotormagneten 31 kann der Motor sehr effizient betrieben werden, so dass sowohl das Motorgehäuse 43 als auch ein gegebenenfalls mit diesem verbundener Griff oder Kühlkörper an seinen berührbaren Oberflächen auf weniger als 40 °C erwärmt wird, wenn die Pumpenanordnung 1 bei einer Drehzahl von 32.000 Umdrehungen pro Minute und einer Förderleistung von 2.5 l pro Minute betrieben wird.

Der in Fig. 6 dargestellte Motor 7' unterscheidet sich von dem in Fig. 6 dargestellten Motor 7 lediglich dadurch, dass der Stator 36 in dieser Ausführung eine flüssigkeitsdichte Hülse 44 aufweist, die den Spalt 40 begrenzt. In dieser Ausführung beträgt die Breite des Spalts 40 0,15 mm. Die Hülse 44 umfasst Polyetheretherketon und ist magnetisch inaktiv. Die Hülse 44 ist derart angeordnet, dass beispielsweise die Wicklungen 37 und weitere Teile des Stators 36 durch die Hülse 44 von der gegebenenfalls durch den Spalt 40 fließenden Spülflüssigkeit getrennt werden. Eine Ausdehnung der Hülse 44 in axialer Richtung beträgt etwa das 1,2-fache einer axialen Ausdehnung des Rotormagneten 31.

Lediglich in den Ausführungsbeispielen offenbarte Merkmale der verschiedenen Ausführungsformen können miteinander kombiniert und einzeln beansprucht werden.

## Patentansprüche

1. Blutpumpe, umfassend eine in einem Katheter (2) geführte und flexible Antriebswelle (3), ein in einem distalen Bereich der Antriebswelle (3) mit der Antriebswelle (3) verbundenes Förderelement (6) und einen Motor (7), wobei der Motor (7) einen Stator (36) und einen in dem Stator (36) drehbar gelagerten Rotor (30) umfasst, wobei der Stator (36) eine Wicklung (37) und der Rotor (30) einen Rotormagneten (31) umfasst, wobei die Antriebswelle (3) an einem proximalen Ende der Antriebswelle (3) mit dem Rotor (30) verbunden ist,
**dadurch gekennzeichnet, dass**
der Stator (36) und der Rotor (30) unlösbar miteinander verbunden sind und einen Spalt (40) ausbilden, der durch den Rotor (30) und den Stator (36) begrenzt wird.

2. Blutpumpe nach Anspruch 1, **dadurch gekennzeichnet, dass** der Spalt (40) eine Breite von maximal 1 mm, vorzugsweise maximal 0,5 mm, besonders vorzugsweise maximal 0,25 mm, aufweist.

3. Blutpumpe nach einem der Ansprüche 1 oder 2, **gekennzeichnet durch** eine Spülöffnung (41), die in fluider Verbindung mit dem Spalt (40) steht.

4. Blutpumpe nach Anspruch 3, **dadurch gekennzeichnet, dass** die Spülöffnung (41) mit einem Spülanschluss (42) verbunden ist, der an einem proximalen Ende des Motors (7) angeordnet ist.

5. Blutpumpe nach einem der Ansprüche 3 oder 4, **dadurch gekennzeichnet, dass** der Spalt (40) in fluider Verbindung mit einem zwischen dem Katheter (2) und der Antriebswelle (3) ausgebildeten Zwischenraum steht.

6. Blutpumpe nach einem der Ansprüche 1 bis 5, **dadurch gekennzeichnet, dass** der Spalt (40) eine minimale Breite von 0,05 mm aufweist.

7. Blutpumpe nach einem der Ansprüche 1 bis 6, **dadurch gekennzeichnet, dass** die Wicklung (37) einen Innenradius aufweist, der maximal einem 1,5-fachen, bevorzugt maximal einem 1,25-fachen, besonders bevorzugt maximal einem 1,15-fachen, eines Außenradius des Rotormagneten (31) entspricht.

8. Blutpumpe nach einem der vorhergehenden Ansprüche, **dadurch gekennzeichnet, dass** ein radialer Abstand zwischen der Wicklung (37) und dem Rotormagneten (31) maximal 2 mm, vorzugsweise maximal 1,25 mm, besonders vorzugsweise maximal 0,75 mm, beträgt.

9. Blutpumpe nach einem der Ansprüche 1 bis 8, **dadurch gekennzeichnet, dass** die Wicklung (37) in eine biokompatible Vergussmasse eingegossen ist.

10. Blutpumpe nach einem der Ansprüche 1 bis 9, **dadurch gekennzeichnet, dass** der Stator (36) eine fluiddichte Hülse (44) mit im Wesentlichen ringförmigem Querschnitt aufweist, durch die der Spalt (40) begrenzt wird.

11. Blutpumpe nach Anspruch 10, **dadurch gekennzeichnet, dass** die Hülse (44) eine Ausdehnung in axialer Richtung aufweist, die geringer als das 1,5-fache einer axialen Ausdehnung des Rotormagneten (31) ist.

12. Blutpumpe nach einem der Ansprüche 1 bis 11, **dadurch gekennzeichnet, dass** der Rotor (30) durch mindestens ein Gleitlager radial gelagert ist.

13. Blutpumpe nach einem der Ansprüche 1 bis 12, **dadurch gekennzeichnet, dass** der Rotor (30) durch mindestens ein Kugellager radial gelagert ist.

14. Blutpumpe nach einem der Ansprüche 1 bis 13, **dadurch gekennzeichnet, dass** das mindestens eine Kugellager nichtmagnetisierbares Material umfasst.

15. Blutpumpe nach einem der Ansprüche 11 oder 14, **dadurch gekennzeichnet, dass** das mindestens eine Kugellager Kugeln aufweist, die ein keramisches Material umfassen.

16. Blutpumpe nach einem der Ansprüche 11 bis 15, **dadurch gekennzeichnet, dass** das mindestens eine Kugellager einen Käfig aufweist, der einen Kunststoff umfasst.

17. Blutpumpe nach einem der Ansprüche 1 bis 16, **dadurch gekennzeichnet, dass** der Rotor (30) eine Beschichtung und/oder eine Abdeckung zum Schutz des Rotormagneten (31) aufweist.

18. Blutpumpe nach einem der Ansprüche 1 bis 17, **dadurch gekennzeichnet, dass** die Blutpumpe eingerichtet ist zum Pumpen von Blut von einem Ventrikel in ein Blutgefäß eines Patienten, wenn der Motor (7) außerhalb eines Körpers des Patienten angeordnet ist.

19. Blutpumpe nach einem der Ansprüche 1 bis 18, **gekennzeichnet durch** einen entfaltbaren Pumpenkopf (4), der das Förderelement (6) und ein Gehäuse (5) umfasst, wobei das Förderelement (6) und das Gehäuse (5) derart ausgebildet sind, dass diese sich nach einer aufgezwungenen Kompression selbstständig entfalten.

20. Betriebsverfahren für eine Blutpumpe nach einem der Ansprüche 1 bis 19, **dadurch gekennzeichnet, dass** sich berührbare Oberflächen eines Gehäuses (43) des Motors (7) während eines Permanentbetriebs bei einer Drehzahl von 15.000, bevorzugt mindestens 30.000, Umdrehungen pro Minute auf eine Temperatur von nicht mehr als 60 °C, vorzugsweise von nicht mehr als 48 °C, besonders vorzugsweise von nicht mehr als 43 °C, erwärmt.
